# EUROPEAN PATENT SPECIFICATION

(11) **EP 3 892 210 B1**
(45) Date of publication and mention of the grant of the patent: **01.05.2024**
(21) Application number: 19893093.5
(22) Date of filing: 05.11.2019
(51) Int. Cl.: A61B 17/22, A61B 17/3207

(54) **THROMBUS EXTRACTION DEVICE**
THROMBUSEXTRAKTIONSVORRICHTUNG
DISPOSITIF D'EXTRACTION DE THROMBUS

(30) Priority: 05.12.2018 CN 201811481427
(43) Date of publication of application: 13.10.2021
(73) Proprietor: Shanghai Bluevascular Medtech Co., Ltd., Shanghai 201318 (CN)
(72) Inventor: SHI, Zengzuo, Shanghai 201318 (CN); HAN, Jianchao, Shanghai 201318 (CN); FAN, Yaming, Shanghai 201318 (CN); ZHANG, Linlin, Shanghai 201318 (CN); ZHU, Qing, Shanghai 201318 (CN); MIAO, Zhenghua, Shanghai 201318 (CN)
(74) Representative: Marks & Clerk LLP
(86) International application number: PCT/CN2019/115807
(87) International publication number: WO 2020/114180

(56) References cited:
- WO-A1-00/78230
- US-A- 4 696 667
- US-A- 5 074 841
- US-A1- 2002 198 550
- US-A1- 2009 099 581
- US-A1- 2012 239 064

## Description

### TECHNICAL FIELD

The present disclosure relates to the technical field of medical devices, and in particular to a thrombus extraction device.

### BACKGROUND

Venous thrombosis is a disease caused by abnormal blood clotting in veins. Venous thrombosis can cause increased venous pressure, obstruction of blood return, swelling, pain and dysfunction of the limbs. In severe cases, post-thrombotic syndrome may be formed, endangering life and safety. Conventionally, thrombus removal has been performed through thrombolysis by intubation or conventional surgical thrombus extraction. However, thrombolysis by intubation requires a long time and is not suitable for patients with a high risk of bleeding. Whereas conventional surgical thrombus extraction is an invasive operation, which may easily damage the function of vascular valves, and is prone to wound complications.

In recent years, mechanical thrombus removal devices have appeared, which utilize methods like dissolution, smashing, and suction to remove thrombus in blood vessels, so as to restore blood circulation and valve function. However, the general mechanical thrombus removal device can only reach areas in blood vessels with the same diameter as the catheter, and areas in the blood vessels with diameters less than the catheter diameter cannot be reached, resulting in that fail to remove the thrombus in blood vessels with small diameters. In addition, when the thrombus adheres to a vascular wall, the general mechanical thrombus removal device cannot suck it all away, resulting in the thrombus still remaining on the vascular wall. US2012239064A1 discloses a medical device for removing a material from a hollow anatomical structure. The device may be used in combination with a distal occlusion element, which may be either a radially expandable filter or balloon member. US2002198550A1 discloses a system and method for treating diseased target tissue of a living being. The system comprises a working head and a debris extraction sub-system. The debris extraction sub-system introduces an infusate liquid at a first flow rate adjacent the working head and withdraws that liquid and some blood at a second and higher flow rate, to create a differential flow adjacent the working head. US2009099581A1 discloses methods and apparatus for treating a vascular occlusion.

### SUMMARY

The invention is defined by independent claim 1. Additional embodiments are defined by the dependent claims.

According to various exemplary embodiments of the present disclosure, a thrombus extraction device is provided.

One aspect of the present application provides a thrombus extraction device, which is configured to remove thrombus along a guide wire. The thrombus extraction device includes:
a thrombus suction assembly including an outer tube, a transmission tube, a driving member, and a thrombus suction head, wherein the outer tube is sleeved outside the transmission tube, one end of the outer tube is connected to the thrombus suction head, the thrombus suction head is provided with a thrombus smashing cutter therein, the thrombus suction head is configured to suck in and smash the thrombus, both ends of the transmission tube are connected to the thrombus smashing cutter and the driving member, respectively, the driving member is configured to drive the transmission tube to rotate; and,
a thrombus extraction assembly configured to draw the thrombus to the thrombus suction head for sucking in and smashing the thrombus by the thrombus suction head, the thrombus extraction assembly including a multi-lumen tube and a balloon provided at one end of the multi-lumen tube, wherein the balloon has a deflated state and a liquid filled inflated state, the multi-lumen tube is provided with a liquid filling cavity therein configured to fill the balloon with liquid and a guide wire cavity therein configured to allow the guide wire to extend through, the multi-lumen tube extends through the transmission tube and the thrombus suction head sequentially, such that the thrombus extraction assembly is movable with respect to the thrombus suction assembly in an axial direction wherein the thrombus suction head is of a hollow structure, the thrombus smashing cutter is provided in the thrombus suction head and is sleeved outside the multi-lumen tube, an end surface of the thrombus suction head is provided with a thrombus pulling hole configured to allow the multi-lumen tube to extend through and at least one thrombus extraction hole configured to suck in the thrombus, an end surface of the thrombus smashing cutter is provided with a blade, and the thrombus extraction hole faces the blade.

The thrombus extraction assembly extends through the above-mentioned thrombus extraction device, and the thrombus extraction assembly is movable with respect to the axial direction of the thrombus suction assembly, such that in some areas of blood vessels with small diameter that cannot be reached by the outer tube of the thrombus suction assembly, the thrombus extraction assembly can be moved to extend through the thrombus, and then the balloon is inflated by filling the balloon with liquid, such that pulling the thrombus extraction assembly can draw the thrombus to the vicinity of the thrombus suction assembly, and the thrombus suction assembly can suck in and smash the thrombus, therefore removing the thrombus. In addition, the balloon can be closely attached to the inner wall of the blood vessels after being inflated, such that when it is pulled, the balloon can remove all the thrombus adhering to the blood vessel wall.

In one of the embodiments, the thrombus extraction assembly further includes a first connecting member, the first connecting member is sleeved on an end of the multi-lumen tube away from the balloon, the first connecting member is provided with a liquid filling port in communication with the liquid filling cavity, and a guide wire port in communication with the guide wire cavity.

In one of the embodiments, the thrombus extraction assembly further includes a tapered head connected to an end of the multi-lumen tube away from the first connecting member, the tapered head has an internal cavity in communication with the guide wire cavity, such that the guide wire can extend out from the tapered head.

In one of the embodiments, a diameter of the tapered head gradually increases in a direction toward the first connecting member.

In one of the embodiments, in the deflated state, the balloon is attached to a surface of the multi-lumen tube.

In one of the embodiments, in the liquid filled inflated state, a volume of the balloon increases, such that a surface of the balloon is closely attached to an inner wall of a blood vessel.

In one of the embodiments, the balloon is sleeved outside the multi-lumen tube, and a sidewall of the multi-lumen tube is provided with a balloon liquid filling port in communication with the liquid filling cavity and an interior of the balloon.

In one of the embodiments, a gap is formed between the outer tube and the transmission tube, and the gap is configured to discharge the thrombus sucked in and smashed by the thrombus suction assembly.

In one of the embodiments, the thrombus extraction assembly further includes a developing wire provided outside the multi-lumen tube and located at both ends of the balloon.

In one of the embodiments, the thrombus suction assembly further includes a second connecting member sleeved on an end of the outer tube away from the thrombus suction head, the second connecting member is provided with a thrombus suction port and a transmission tube port, the thrombus suction port is in communication with an interior of the outer tube, and the transmission tube port is configured to allow the transmission tube to extend through.

In one of the embodiments, the thrombus suction assembly further includes a negative pressure suction device, the negative pressure suction device is provided with a suction port in communication with the thrombus suction port.

In one of the embodiments, the negative pressure suction device is further provided with a discharge port connected to a collection bag.

In one of the embodiments, the thrombus pulling hole is provided at a center of the end surface of the thrombus suction head.

In one of the embodiments, there are a plurality of the thrombus extraction holes, and the plurality of thrombus extraction holes are evenly provided along a circumferential direction of the thrombus pulling hole.

In one of the embodiments, the thrombus smashing cutter is provided with a center through hole in communication with the thrombus pulling hole; a diameter of the multi-lumen tube is less than a diameter of the transmission tube, a diameter of the thrombus pulling hole, a diameter of the center through hole.

In one of the embodiments, there are a plurality of sets of the thrombus extraction assemblies, and the plurality of sets of the thrombus extraction assemblies extend through the transmission tube and the thrombus suction head, and each set of the thrombus extraction assemblies is movable with respect to the thrombus suction assembly, and the plurality of sets of the thrombus extraction assemblies are sleeved outside different guide wires, respectively.

One aspect of the disclosure not covered by the claims also provides a thrombus extraction method utilizing any of the above-mentioned thrombus extraction devices. The method includes:
allowing one end of the guide wire to extend into the blood vessel and through the thrombus, and allowing the other end of the guide wire to extend into the guide wire cavity of the thrombus extraction device;
feeding the thrombus extraction device into the blood vessel along the guide wire, and after reaching the stenosis of the blood vessel, the thrombus suction assembly is kept still;
pushing the thrombus extraction assembly to continue to move forward along the guide wire to extend through the thrombus;
filling the balloon with liquid through the multi-lumen tube, so that the balloon changes from the deflated state to the liquid filled inflated state;
pulling the thrombus extraction assembly toward a direction of the thrombus suction head, drawing the thrombus to the vicinity of the thrombus suction head; and
the thrombus suction head sucking in the nearby thrombus, smashing the thrombus with the thrombus smashing cutter, and discharging the smashed thrombus.

### BRIEF DESCRIPTION OF THE DRAWINGS

In order to clearly describe the technical solutions in the present embodiments or in the prior art, the drawings needed to be used in the description of the embodiments or the prior art will be briefly introduced in the following. It is clear that, the drawings in the following description are merely some of the embodiments of the present application, for those of ordinary skills in the art, without creative work, the drawings of other embodiments can also be obtained based on these drawings.
FIG. 1 is a structural schematic view of a thrombus extraction device according to an embodiment of the present application;
FIG. 2 is a cross-sectional view taken along an A-A section of the thrombus extraction device shown in FIG. 1;
FIG. 3 is a structural schematic view of a thrombus extraction assembly of the thrombus extraction device shown in FIG. 1;
FIG. 4 is a schematic view of a balloon of the thrombus extraction assembly shown in FIG. 3 in a deflated state;
FIG. 5 is a schematic view of the balloon of the thrombus extraction assembly shown in FIG. 3 in an inflated state;
FIG. 6 is a structural schematic view of a thrombus suction head according to an embodiment of the present disclosure but that is not within the scope of the present invention;
FIG. 7 is a structural schematic view of a thrombus suction head according to an embodiment of the present invention;
FIG. 8 is a schematic view of a thrombus extraction device of an embodiment of the present application in a using state;
FIG. 9 is a schematic view of a thrombus extraction device of another embodiment of the present application in a using state;
FIG. 10 is a flowchart of a thrombus extraction method of an embodiment of the present disclosure.

### DETAILED DESCRIPTION OF THE EMBODIMENTS

In order to make the above objectives, features and advantages of the present application more obvious and understandable, the specific implementations of the present application will be described in detail below with reference to the accompanying drawings. In the following description, many specific details are set forth in order to fully understand this application. However, this application can be implemented in many other ways different from those described herein, and those skilled in the art can make similar improvements without violating the connotation of this application. Therefore, this application is not limited by the specific implementation disclosed below

It should be noted that when an element is referred to as being "fixed to" another element, it can be directly on the other element or an intermediate element may also be present. When an element is considered to be "connected" to another element, it can be directly connected to the other element or an intermediate element may be present at the same time.

Unless otherwise defined, all technical and scientific terms used herein have the same meaning as commonly understood by those skilled in the technical field of this application. The terms used in the specification of the application herein are only for the purpose of describing specific embodiments, and are not intended to limit the application. The term "and/or" as used herein includes any and all combinations of one or more related listed items.

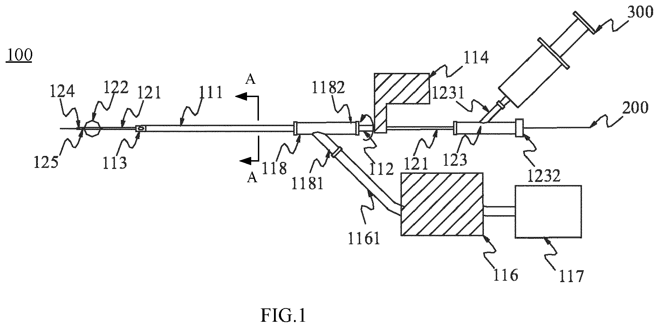

Referring to FIGs. 1 to 8, an embodiment of the present application provides a thrombus extraction device 100, which is configured to remove thrombus along a guide wire 200, especially to remove thrombus in areas of blood vessels with smaller diameters. Specifically, the thrombus extraction device 100 of an embodiment includes a thrombus suction assembly for sucking, smashing, and discharging the thrombus, and a thrombus extraction assembly for drawing the thrombus from the blood vessels to the vicinity of the thrombus suction assembly along the guide wire 200. The guide wire 200 used herein is a metal wire that is pre-implanted in the blood vessels and passing through the thrombus before the thrombus extraction operation, and is configured to guide the thrombus extraction device 100.

Specifically, the thrombus suction assembly includes an outer tube 111, a transmission tube 112, a thrombus suction head 113, and a driving member 114. The outer tube 111 is sleeved outside the transmission tube 112, and a gap 1111 is formed between the outer tube 111 and the transmission tube 112. The thrombus can be discharged through the gap 1111 after being sucked in and smashed. Further, one end of the outer tube 111 is connected to the thrombus suction head 113, and the thrombus suction head 113 is configured to suck in and smash the thrombus. The thrombus suction head 113 is provided with a thrombus smashing cutter 115 therein configured to smash thrombus. Both ends of the transmission tube 112 are connected to the thrombus smashing cutter 115 and the driving member 114, respectively, so as to transmit a torque of the driving member 114 to the thrombus smashing cutter 115, and drive the thrombus smashing cutter 115 to rotate. In one embodiment, the driving member 114 may be a rotary motor. The material of the thrombus suction head 113 may be a metal material or a composite material of medical polymer and metal. The material of the outer tube 111 may be a medical polymer material or a composite material of medical polymer and metal. The material of the transmission tube 112 may be a metal material or a composite material of medical polymer and metal.

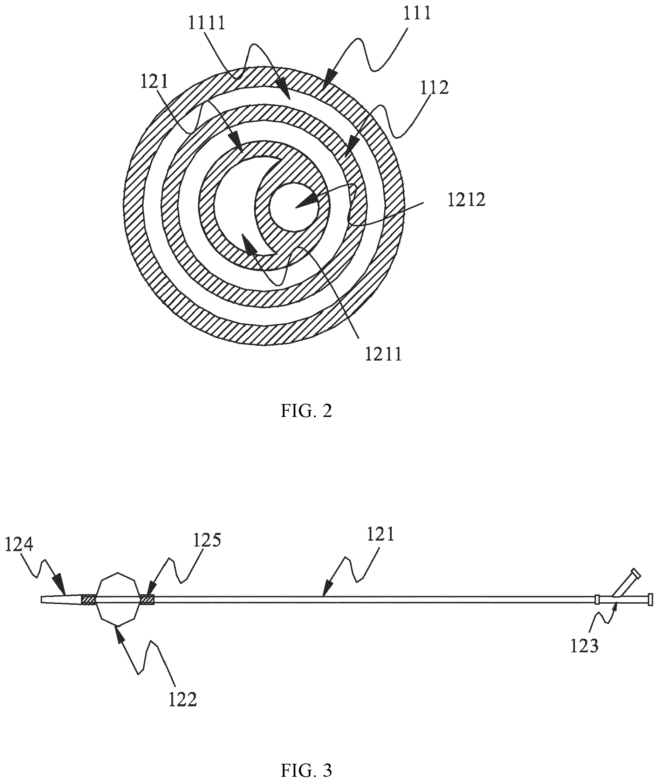

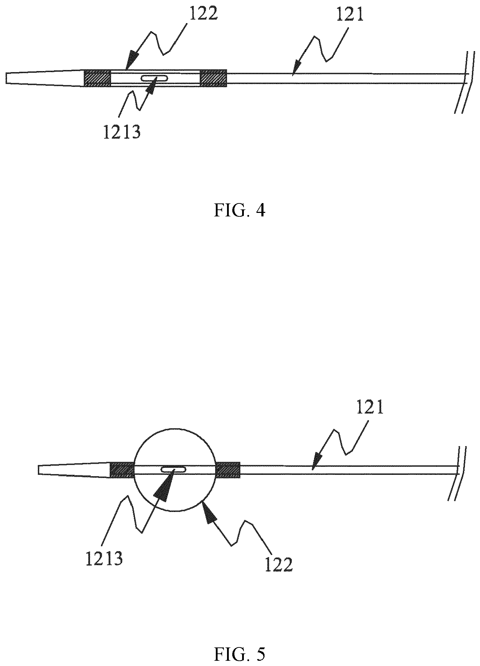

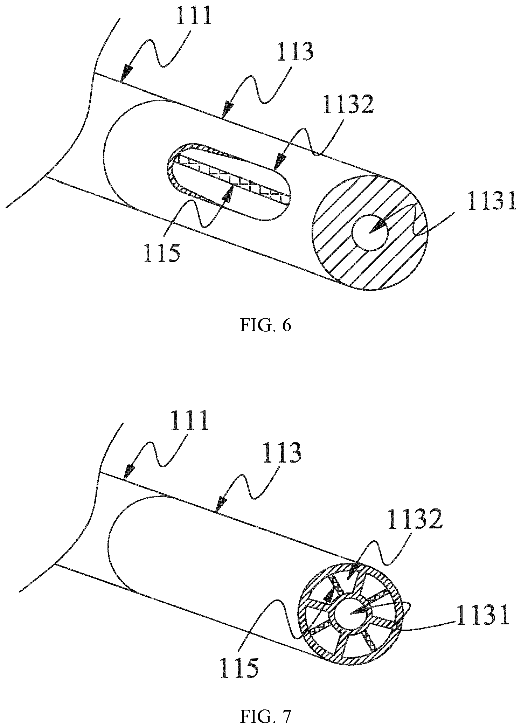

Further, the thrombus extraction assembly extends through the thrombus suction assembly along an axial direction of the thrombus suction assembly, and is movable with respect to the thrombus suction assembly in the axial direction. Specifically, the thrombus extraction assembly includes a balloon 122 and a multi-lumen tube 121 connected to the balloon 122. The balloon 122 can deflate or inflate by filling with liquid. In other words, the balloon 122 has a deflated state (as shown in FIG. 4) and an inflated state after being filled with liquid (as shown in FIG. 5). In the deflated state, the balloon 122 is attached on the surface of the multi-lumen tube 121. In the inflated state, the volume of the balloon 122 increases, such that the surface of the balloon 122 is closely attached to an inner wall of the blood vessels. Specifically, referring to FIG. 2, the multi-lumen tube 121 is provided with a liquid filling cavity 1211 therein configured to fill the balloon 122 with liquid, and a guide wire cavity 1212 configured to allow the guide wire 200 to extend through. A diameter of the multi-lumen tube 121 is less than a diameter of the transmission tube 112 of the thrombus suction assembly, is less than a diameter of a thrombus pulling hole 1131 of the thrombus suction head 113 (refer to FIG. 6), and is less than a diameter of a center through hole of the thrombus smashing cutter 115 in the thrombus suction head 113 (not shown), such that the multi-lumen tube 121 can extend through the transmission tube 112 and the thrombus suction head 113 sequentially. Therefore, in some areas of the blood vessels with small diameter that cannot be reached by the thrombus suction assembly, doctors can push the multi-lumen tube 121 to move the thrombus extraction assembly with respect to the thrombus suction assembly, such that the thrombus extraction assembly can continue to advance along the guide wire 200 and extend through the thrombus. The balloon is inflated by filling the balloon with liquid, then the multi-lumen tube 121 is pulled to pull the thrombus extraction assembly back, and the thrombus in areas of the blood vessels with small diameter can be drawn to the vicinity of the thrombus suction head 113 for suction and smashing by the thrombus suction head 113. In one embodiment, a material of the balloon 122 is medical silica gel, latex or elastomeric polymer material. A material of the multi-lumen tube 121 is a medical polymer material or a composite material of medical polymer and metal. Further, the multi-lumen tube 121 may be a flexible tube, so that it can bend following a trend of the blood vessels.

A thrombus extraction assembly extends through the thrombus suction assembly of the above-mentioned thrombus extraction device 100, and the thrombus extraction assembly is movable in the axial direction with respect to the thrombus suction assembly. In some areas of the blood vessels with small diameter where the outer tube 111 of the thrombus suction assembly cannot reach, the thrombus extraction assembly can enter the areas of the blood vessels with small diameter and extend through the thrombus by moving the multi-lumen tube 121. Then, the balloon 122 is inflated by filling the balloon 122 with liquid, and the multi-lumen tube 121 is pulled so that the thrombus can be drawn to the vicinity of the thrombus suction assembly. The thrombus suction assembly can suck in and smash the thrombus, thus removing the thrombus. In addition, the balloon 122 can be closely attached to the inner wall of the blood vessels after being inflated, so that when the multi-lumen tube 121 is pulled, the balloon 122 can remove all the thrombus adhering to the blood vessel wall.

Referring to FIGS. 1 and 2, in one of the embodiments, the thrombus extraction assembly further includes a first connecting member 123, which is sleeved on an end of the multi-lumen tube 121 away from the balloon 122. Further, the first connecting member 123 is provided with a liquid filling port 1231 and a guide wire port 1232. Specifically, the liquid filling port 1231 is in communication with a liquid filling cavity 1211 of the multi-lumen tube 121 and is configured to inject liquid into the liquid filling cavity 1211, so as to inflate the balloon 122. Furthermore, a syringe 300 can be connected to the liquid filling port 1231 to facilitate the injection of liquid into the liquid filling cavity 1211. The guide wire port 1232 is in communication with the guide wire cavity 1212, and the guide wire port 1232 is configured to allow the guide wire 200 to extend through. In one embodiment, the first connecting member 123 is made of a medical polymer material.

Referring to FIGS. 2 and 3, in one of the embodiments, the thrombus extraction assembly further includes a tapered head 124, which is connected to one end of the multi-lumen tube 121 away from the first connecting member 123. Specifically, the tapered head 124 has a hollow structure, and an internal cavity of the tapered head 124 is in communication with the guide wire cavity 1212, so that the guide wire 200 can extend through the tapered head 124. Further, a diameter of the tapered head 124 gradually increases along a direction toward the first connecting member 123, which makes it easier to extend through the thrombus and prevent damage to the inner wall of the blood vessels. Preferably, the tapered head 124 is made of a medical polymer material.

Referring to FIGS. 3 and 4, in one of the embodiments, the balloon 122 is sleeved outside the multi-lumen tube 121, and the side wall of the multi-lumen tube 121 is provided with a balloon liquid filling port 1213 in communication with the liquid filling cavity 1211 and an interior of the balloon 122. The balloon liquid filling port 1213 is configured to fill the balloon 122 with liquid. Furthermore, the thrombus extraction assembly further includes a developing wire 125, which is provided outside the multi-lumen tube 121 and located at both ends of the balloon 122 in the axial direction. The developing wire 125 has developing properties, so that a position of the balloon 122 can be obtained by a medical imaging device during the thrombus extraction process. In one embodiment, the developing wire 125 is made of a medical polymer material or a metal material.

Referring to FIG. 1, specifically, the thrombus suction assembly further includes a second connecting member 118, which is sleeved on an end of the outer tube 111 away from the thrombus suction head 113. The second connecting member 118 is provided with a thrombus suction port 1181 and a transmission tube port 1182. The thrombus suction port 1181 is in communication with an interior of the outer tube 111, so that the thrombus between the outer tube 111 and the transmission tube 112 can be discharged from the thrombus suction port 1181. The transmission tube port 1182 is configured to allow the transmission tube 112 to extend through.

Further, a negative pressure suction device 116 is connected to the thrombus suction port 1181 to provide negative pressure to suck out the thrombus. Specifically, a suction port of the negative pressure suction device 116 is in communication with the thrombus suction port 1181 through a hose 1161. A discharge port of the negative pressure suction device 116 is configured to connect to the collection bag 117 to collect the thrombus. In one embodiment, the negative pressure suction device 116 may be a negative pressure pump.

Referring to FIG. 6, the thrombus suction head 113 of an embodiment of the present disclosure but that is not encompassed by the present invention has a hollow structure. An end surface of the thrombus suction head 113 is provided with a thrombus pulling hole 1131 configured to allow the multi-lumen tube 121 to extend through, and the multi-lumen tube 121 extends out from the thrombus pulling hole 1131. Furthermore, a side surface of the thrombus suction head 113 is provided with at least one thrombus extraction hole 1132, and the thrombus extraction hole 1132 is configured to suck in the thrombus. The thrombus smashing cutter 115 is provided in the thrombus suction head 113 and is sleeved outside the multi-lumen tube 121, and is connected to the transmission tube 112. Specifically, the thrombus smashing cutter 115 is provided with a center through hole (not shown) in communication with the thrombus pulling hole 1131, and the multi-lumen tube 121 extends through the center through hole and the thrombus pulling hole 1131 sequentially, and extends out from the thrombus suction head 113. A side surface of the thrombus smashing cutter 115 is provided with a blade, and the thrombus extraction hole 1132 faces the blade, so that the thrombus can be smashed by the blade after entering the thrombus extraction hole 1132.

Referring to FIG. 7, in an embodiment according to the invention, the thrombus suction head 113 has a hollow structure, and the end surface of the thrombus suction head 113 is provided with a thrombus pulling hole 1131 configured to allow the multi-lumen tube 121 to extend through, and a thrombus extraction hole 1132 configured to suck in the thrombus. The thrombus smashing cutter 115 is provided in the thrombus suction head 113 and sleeved outside the multi-lumen tube 121. Specifically, the thrombus smashing cutter 115 is provided with the center through hole (not shown) in communication with the thrombus pulling hole 1131. The multi-lumen tube 121 extends through the center through hole and the thrombus pulling hole 1131 sequentially, and extends out from the thrombus suction head 113. An end surface of the thrombus smashing cutter 115 is provided with a blade, and the thrombus extraction hole 1132 faces the blade. Further, the end surface of the thrombus suction head 113 can be directly integrally formed with a side peripheral surface of the thrombus suction head 113, or a distal end of the thrombus suction head 113 of a hollow cylindrical shape can be covered with a hollow baffle to form the aforementioned end surface that is provided with the thrombus pulling hole 1131 and the thrombus extraction hole 1132.

Further, referring to FIG. 7 again, in this embodiment, the thrombus pulling hole 1131 is provided in a center of the end surface of the thrombus suction head 113, and the multi-lumen tube 121 extends out from the thrombus pulling hole 1131. There is a plurality of the thrombus extraction holes 1132, and the plurality of the thrombus extraction holes 1132 are evenly provided along a circumferential direction of the thrombus pulling hole 1131. The plurality of the thrombus extraction holes 1132 can speed up the suction of the thrombus.

Please continue to refer to FIGS. 1 to 8, during operation, the blood vessel 400 is firstly punctured, and then one end of the guide wire 200 extends into the diseased blood vessel and through the thrombus 410, while the other end of the guide wire 200 extends into the guide wire cavity 1212 of the thrombus extraction device 100. A front end of the thrombus extraction device 100 is fed into the blood vessel along the guide wire 200, and after a stenosis of the blood vessel 400 is reached, the thrombus suction assembly is kept still, and by pushing the first connecting member 123, the thrombus extraction assembly is continued to move along the guide wire 200 and extends through the diseased thrombus 410. Then, the syringe 300 is connected to the liquid filling port 1231, and the balloon 122 is inflated with iopamidol until the balloon 122 is attached to the blood vessel wall. By pulling the first connecting member 123, the thrombus extraction assembly moves toward the thrombus suction head 113, meanwhile the driving member 114 and the negative pressure suction device 116 of the thrombus suction assembly are turned on. The thrombus is smashed by the thrombus smashing cutter 115 in the thrombus suction head 113, meanwhile the thrombus is discharged into the collection bag 117 by the negative pressure generated by the negative pressure suction device 116. When the thrombus is removed, the balloon 122 can be deflated, and it can return to the deflated state, and the driving member 114 and the negative pressure suction device 116 are turned off. After the thrombus extraction is completed, the thrombus extraction device 100 is withdrawn from the body.

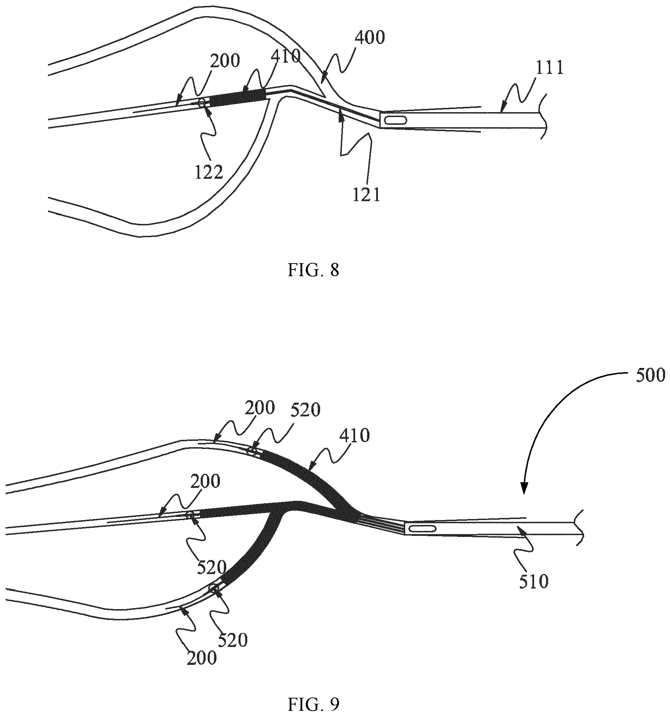

Referring to FIG. 9, in another embodiment, one set of the thrombus suction assemblies may also be equipped with a plurality of sets of the thrombus extraction assemblies, so that the thrombus in cavities of a plurality of branch blood vessels can be removed simultaneously. Specifically, a thrombus extraction device 500 of an embodiment is configured to remove thrombus in different blood vessels along a plurality of guide wires 200 respectively. The thrombus extraction device 500 includes a thrombus suction assembly 510 and a plurality of sets of thrombus suction assemblies 520. The thrombus suction assembly 510 is similar in structure to the thrombus extraction assembly described in the aforementioned embodiments, and includes an outer tube, a transmission tube, a thrombus suction head, and a driving member. The outer tube is sleeved outside the transmission tube, and a gap is formed between the outer tube and the transmission tube, through which the thrombus can be discharged after being sucked in and smashed. Further, one end of the outer tube is connected to the thrombus suction head, the thrombus suction head is configured to suck in and smash the thrombus. A thrombus smashing cutter is provided in the thrombus suction head configured to smash the thrombus. Both ends of the transmission tube are connected to the thrombus smashing cutter and the driving member respectively, so as to transmit a torque of the driving member to the thrombus smashing cutter to drive the thrombus smashing cutter to rotate. In one embodiment, the driving member may be a rotary motor, the thrombus suction head may be a metal or a composite material of medical polymer and metal, the outer tube is a medical polymer material or a composite material of medical polymer and metal, and the transmission tube is of a metal material or a composite material of medical polymer and metal.

Further, the plurality of sets of the thrombus extraction assemblies 520 extend through the transmission tube and the driving member, and each thrombus extraction assembly 520 is movable with respect to the thrombus suction assembly 510. Specifically, each thrombus extraction assembly 520 includes a balloon and a multi-lumen tube connected to the balloon, wherein the balloon can be deflated or inflated by filling with liquid. In other words, the balloon has a deflated state and an inflated state after being filled with liquid. In the deflated state, the balloon can attach on the surface of the multi-lumen tube. In the inflated state, the volume of the balloon can increase, such that the surface of the balloon attaches closely to the inner wall of the blood vessel. The multi-lumen tube is provided with a liquid filling cavity for filling the balloon with liquid, and a guide wire cavity for allowing a guide wire to extend through. Specifically, the guide wire cavity of each multi-lumen tube is respectively configured to allow different guide wires to extend through, so that the plurality of sets of the thrombus extraction assemblies 520 can respectively be moved along different guide wires to different blood vessel branches, such that the thrombus in different blood vessel branches is drawn to the vicinity of the thrombus suction head.

It should be noted that, if there is no contradiction, the other features of the thrombus suction assembly 510 and each set of the thrombus extraction assemblies 520 can be configured in the same manner as the previous embodiment, and will not be repeated here.

The plurality of sets of the thrombus extraction assemblies 520 extend through the thrombus suction assembly 510 of the above-mentioned thrombus extraction device 500, and each set of the thrombus extraction assemblies 520 is movable with respect to the thrombus suction assembly 510 in an axial direction of the thrombus suction assembly 510. In some multi-branched small-diameter blood vessel areas that cannot be reached by the outer tube of the thrombus suction assembly 510, the plurality of sets of the thrombus extraction assemblies 520 can be moved to different branch blood vessels along different guide wires. Then, through the balloon that is inflatable by filling with liquid, the thrombus in different branch blood vessels is drawn to the vicinity of the thrombus suction head, which improves the efficiency of thrombus extraction. In addition, the thrombus suction head can suck in and smash the thrombus, and then remove the thrombus. After the balloon is inflated, it can be closely attached to the inner wall of the blood vessel, so that when it is pulled, the thrombus adhering to the blood vessel wall can be removed completely.

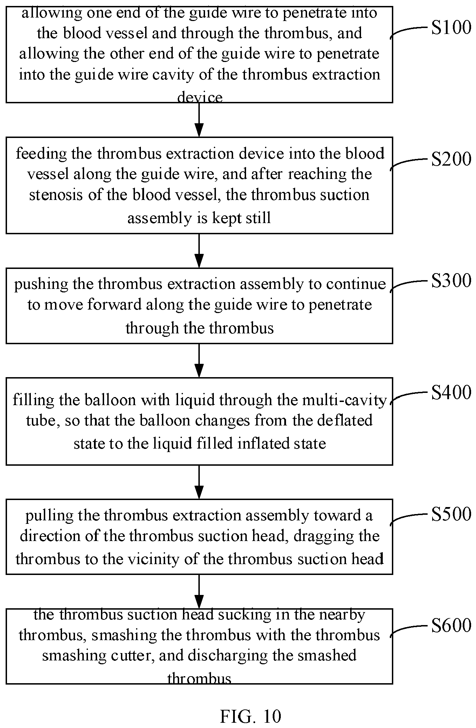

Referring to FIG. 10, an exemplary embodiment of the present disclosure not covered by the claims provides a thrombus extraction method, which is used with the thrombus extraction device in each of the aforementioned embodiments. The thrombus extraction method includes the following steps.

In S 100, one end of the guide wire extends into the blood vessel and through the thrombus, and the other end of the guide wire extends into the guide wire cavity of the thrombus extraction device.

In S200, the thrombus extraction device is fed into the blood vessel along the guide wire, and after reaching the stenosis of the blood vessel, the thrombus suction assembly is kept still.

In S300, the thrombus extraction assembly is pushed to continue to move forward along the guide wire to extend through the thrombus.

In S400, the balloon is filled with liquid through the multi-lumen tube, so that the balloon changes from the deflated state to the liquid filled inflated state.

In S500, the thrombus extraction assembly is pulled in a direction of towards the thrombus suction head, the thrombus is drawn to the vicinity of the thrombus suction head.

In S600, the thrombus suction head sucks in the nearby thrombus, smashes the thrombus with the thrombus smashing cutter, and discharges the smashed thrombus.

The steps in the above method have been described in detail in the embodiments of the thrombus extraction device, and will not be repeated here. It should be understood that although the various steps in the flowchart of FIG. 10 are displayed sequentially as indicated by the arrows, these steps are not necessarily executed in the order indicated by the arrows. Unless there is a clear description herein, there is no strict order for the execution of these steps, and these steps can be executed in other orders. Moreover, at least a part of the steps in FIG. 10 may include a plurality of sub-steps or a plurality of stages. These sub-steps or stages are not necessarily executed at the same time, but can be executed at different times. The execution of these sub-steps or stages is not necessarily performed sequentially, but may be performed by taking turns or alternately with other steps or at least a part of the sub-steps or stages of other steps.

## Claims

1. A thrombus extraction device (100) configured to remove thrombus along a guide wire (200), the thrombus extraction device comprising:
a thrombus suction assembly comprising an outer tube (111), a transmission tube (112), a driving member (114), and a thrombus suction head (113), wherein the outer tube is sleeved outside the transmission tube, one end of the outer tube is connected to the thrombus suction head, the thrombus suction head is provided with a thrombus smashing cutter (115) therein, the thrombus suction head is configured to suck in and smash the thrombus, both ends of the transmission tube are connected to the thrombus smashing cutter and the driving member, respectively, the driving member is configured to drive the transmission tube to rotate; and,
a thrombus extraction assembly configured to draw the thrombus to the thrombus suction head, the thrombus extraction assembly comprising a multi-lumen tube (121) and a balloon (122) provided at one end of the multi-lumen tube, wherein the balloon has a deflated state and a liquid filled inflated state, the multi-lumen tube is provided with a liquid filling cavity (1211) therein configured to fill the balloon with liquid and a guide wire cavity (1212) therein configured to allow the guide wire to extend through, the multi-lumen tube extends through the transmission tube and the thrombus suction head sequentially, such that the thrombus extraction assembly is movable with respect to the thrombus suction assembly in an axial direction;
wherein the thrombus suction head is of a hollow structure, the thrombus smashing cutter is provided in the thrombus suction head and is sleeved outside the multi-lumen tube, an end surface of the thrombus suction head is provided with a thrombus pulling hole (1131) configured to allow the multi-lumen tube to extend through and at least one thrombus extraction hole (1132) configured to suck in the thrombus, an end surface of the thrombus smashing cutter is provided with a blade, and the thrombus extraction hole faces the blade.

2. The thrombus extraction device according to claim 1, wherein the thrombus extraction assembly further comprises a first connecting member (123), the first connecting member is sleeved on an end of the multi-lumen tube (121) away from the balloon (122), the first connecting member is provided with a liquid filling port (1231) in communication with the liquid filling cavity (1211), and a guide wire port (1232) in communication with the guide wire cavity (1212).

3. The thrombus extraction device according to claim 2, wherein the thrombus extraction assembly further comprises a tapered head (124) connected to an end of the multi-lumen tube (121) away from the first connecting member (123), the tapered head has an internal cavity in communication with the guide wire cavity (1212), such that the guide wire (200) can extend out from the tapered head.

4. The thrombus extraction device according to claim 3, wherein a diameter of the tapered head (124) gradually increases in a direction toward the first connecting member (123).

5. The thrombus extraction device according to claim 1, wherein in the deflated state, the balloon (122) is attached to a surface of the multi-lumen tube (121).

6. The thrombus extraction device according to claim 1, wherein in the liquid filled inflated state, a volume of the balloon (122) increases, such that a surface of the balloon is closely attached to an inner wall of a blood vessel.

7. The thrombus extraction device according to claim 1, wherein the balloon (122) is sleeved outside the multi-lumen tube (121), and a sidewall of the multi-lumen tube is provided with a balloon liquid filling port (1213) in communication with the liquid filling cavity (1211) and an interior of the balloon.

8. The thrombus extraction device according to claim 1, wherein a gap (1111) is formed between the outer tube (111) and the transmission tube (112), and the gap is configured to discharge the thrombus sucked in and smashed by the thrombus suction assembly.

9. The thrombus extraction device according to claim 1, wherein the thrombus extraction assembly further comprises a developing wire (125) provided outside the multi-lumen tube (121) and located at both ends of the balloon (122).

10. The thrombus extraction device according to claim 1, wherein the thrombus suction assembly further comprises a second connecting member (118) sleeved on an end of the outer tube (111) away from the thrombus suction head (113), the second connecting member is provided with a thrombus suction port (1181) and a transmission tube port (1182), the thrombus suction port is in communication with an interior of the outer tube, and the transmission tube port is configured to allow the transmission tube to extend through, the thrombus suction assembly further comprises a negative pressure suction device (116), the negative pressure suction device is provided with a suction port (1181) in communication with the thrombus suction port, the negative pressure suction device is further provided with a discharge port connected to a collection bag (117).

11. The thrombus extraction device according to claim 1, wherein the thrombus pulling hole (1131) is provided at a center of the end surface of the thrombus suction head (113).

12. The thrombus extraction device according to claim 11, wherein there are a plurality of the thrombus extraction holes (1132), and the plurality of thrombus extraction holes are evenly provided along a circumferential direction of the thrombus pulling hole (1131).

13. The thrombus extraction device according to claim 1, wherein the thrombus smashing cutter (115) is provided with a center through hole in communication with the thrombus pulling hole (1131); a diameter of the multi-lumen tube (121) is less than a diameter of the transmission tube (112), a diameter of the thrombus pulling hole, a diameter of the center through hole.

14. The thrombus extraction device according to claim 1, wherein there are a plurality of sets of the thrombus extraction assemblies, and the plurality of sets of the thrombus extraction assemblies extend through the transmission tube (112) and the thrombus suction head (113), and each set of the thrombus extraction assemblies is movable with respect to the thrombus suction assembly, and the plurality of sets of the thrombus extraction assemblies are sleeved outside different guide wires (200), respectively.

## Patentansprüche

1. Thrombusextraktionsvorrichtung (100), konfiguriert zum Entfernen eines Thrombus entlang eines Führungsdrahts (200), wobei die Thrombusextraktionsvorrichtung Folgendes umfasst:
eine Thrombusabsauganordnung, umfassend einen äußeren Schlauch (111), einen Übertragungsschlauch (112), ein Antriebselement (114) und einen Thrombusabsaugkopf (113), wobei der äußere Schlauch außerhalb des Übertragungsschlauchs mit einer Hülse versehen ist, ein Ende des äußeren Schlauchs mit dem Thrombusabsaugkopf verbunden ist, der Thrombusabsaugkopf mit einem Thrombuszerkleinerungsschneider (115) darin versehen ist, der Thrombusabsaugkopf konfiguriert ist, um den Thrombus anzusaugen und zu zerkleinern, beide Enden des Übertragungsschlauchs jeweils mit dem Thrombuszerkleinerungsschneider und dem Antriebselement verbunden sind, das Antriebselement konfiguriert ist, um den Übertragungsschlauch anzutreiben, damit er sich dreht; und,
eine Thrombusextraktionsanordnung, die so konfiguriert ist, dass sie den Thrombus zu dem Thrombusabsaugkopf zieht, wobei die Thrombusextraktionsanordnung einen Mehrlumenschlauch (121) und einen Ballon (122) umfasst, der an einem Ende des Mehrlumenschlauchs bereitgestellt ist, wobei der Ballon einen entleerten Zustand und einen flüssigkeitsgefüllten, aufgeblasenen Zustand aufweist, der Mehrlumenschlauch mit einem Flüssigkeitsfüllhohlraum (1211) darin , der konfiguriert ist, um den Ballon mit Flüssigkeit zu füllen, und einem Führungsdrahthohlraum (1212) darin versehen ist, der konfiguriert ist, um zu ermöglichen, dass sich der Führungsdraht hindurch erstreckt, sich der Mehrlumenschlauch durch den Übertragungsschlauch und den Thrombusabsaugkopf sequenziell erstreckt, sodass die Thrombusextraktionsanordnung in Bezug auf die Thrombusabsauganordnung in einer axialen Richtung beweglich ist;
wobei der Thrombusabsaugkopf von hohler Struktur ist, der Thrombuszerkleinerungsschneider in dem Thrombusabsaugkopf bereitgestellt ist und außerhalb des Mehrlumenschlauchs mit einer Hülse versehen ist, eine Endfläche des Thrombusabsaugkopfs mit einem Thrombuszugloch (1131)das so konfiguriert ist, dass sich der Mehrlumenschlauch hindurch erstrecken kann, und mit mindestens einem Thrombusextraktionsloch (1132) versehen ist, das so konfiguriert ist, dass es den Thrombus einsaugt, eine Endfläche des Thrombuszerkleinerungsschneiders mit einer Klinge versehen ist, und das Thrombusextraktionsloch der Klinge zugewandt ist.

2. Thrombusextraktionsvorrichtung nach Anspruch 1, wobei die Thrombusextraktionsanordnung ferner ein erstes Verbindungselement (123) umfasst, das erste Verbindungselement an einem vom Ballon (122) entfernten Ende des Mehrlumenschlauchs (121) mit einer Hülse versehen ist, das erste Verbindungselement mit einem Flüssigkeitsfüllanschluss (1231) in Kommunikation mit dem Flüssigkeitsfüllhohlraum (1211), und einem Führungsdrahtanschluss (1232) in Kommunikation mit dem Führungsdrahthohlraum (1212) versehen ist.

3. Thrombusextraktionsvorrichtung nach Anspruch 2, wobei die Thrombusextraktionsanordnung ferner einen sich verjüngenden Kopf (124) umfasst, der mit einem vom ersten Verbindungselement (123) entfernten Ende des Mehrlumenschlauchs (121) verbunden ist, der sich verjüngende Kopf einen inneren Hohlraum aufweist, der mit dem Führungsdrahthohlraum (1212) in Kommunikation steht, sodass sich der Führungsdraht (200) aus dem sich verjüngenden Kopf heraus erstrecken kann.

4. Thrombusextraktionsvorrichtung nach Anspruch 3, wobei ein Durchmesser des sich verjüngenden Kopfs (124) in einer Richtung zu dem ersten Verbindungselement (123) hin progressiv zunimmt.

5. Thrombusextraktionsvorrichtung nach Anspruch 1, wobei der Ballon (122) im entleerten Zustand an einer Oberfläche des Mehrlumenschlauchs (121) angebracht ist.

6. Thrombusextraktionsvorrichtung nach Anspruch 1, wobei in dem mit Flüssigkeit gefüllten, aufgeblasenen Zustand ein Volumen des Ballons (122) zunimmt, sodass eine Oberfläche des Ballons eng an einer Innenwand eines Blutgefäßes angebracht ist.

7. Thrombusextraktionsvorrichtung nach Anspruch 1, wobei der Ballon (122) außerhalb des Mehrlumenschlauchs (121) mit einer Hülse versehen ist, und eine Seitenwand des Mehrlumenschlauchs mit einem Ballonflüssigkeitsfüllanschluss (1213) in Kommunikation mit dem Flüssigkeitsfüllhohlraum (1211) und einem Inneren des Ballons versehen ist.

8. Thrombusextraktionsvorrichtung nach Anspruch 1, wobei ein Spalt (1111) zwischen dem Außenschlauch (111) und dem Übertragungsschlauch (112) gebildet ist, und der Spalt konfiguriert ist, um den in die Thrombusabsauganordnung eingesaugten und zerkleinerten Thrombus abzulassen.

9. Thrombusextraktionsvorrichtung nach Anspruch 1, wobei die Thrombusextraktionsanordnung ferner einen Entwicklungsdraht (125) umfasst, der außerhalb des Mehrlumenschlauchs (121) bereitgestellt ist und sich an beiden Enden des Ballons (122) befindet.

10. Thrombusextraktionsvorrichtung nach Anspruch 1, wobei die Thrombusabsauganordnung ferner ein zweites Verbindungselement (118) umfasst, das an einem vom Thrombusabsaugkopf (113) entfernten Ende des äußeren Schlauchs (111) mit einer Hülse versehen ist, das zweite Verbindungselement mit einem Thrombusabsauganschluss (1181) und einem Übertragungsschlauchanschluss (1182) versehen ist, der Thrombusabsauganschluss in Kommunikation mit einem Inneren des äußeren Schlauchs steht, und der Übertragungsschlauchanschluss so konfiguriert ist, dass sich der Übertragungsschlauch hindurch erstrecken kann, die Thrombusabsauganordnung ferner eine Unterdruckabsaugvorrichtung (116) umfasst, die Unterdruckabsaugvorrichtung mit einem Sauganschluss (1181) in Kommunikation mit dem Thrombusabsauganschluss versehen ist, die Unterdruckabsaugvorrichtung ferner mit einem Ablassanschluss versehen ist, der mit einem Sammelbeutel (117) verbunden ist.

11. Thrombusextraktionsvorrichtung nach Anspruch 1, wobei das Thrombuszugloch (1131) an einem Mittelpunkt der Endfläche des Thrombusabsaugkopfs (113) bereitgestellt ist.

12. Thrombusextraktionsvorrichtung nach Anspruch 11, wobei eine Vielzahl von Thrombusextraktionslöchern (1132) vorhanden ist, und die Vielzahl von Thrombusextraktionslöchern gleichmäßig entlang einer Umfangsrichtung des Thrombusextraktionslochs (1131) bereitgestellt ist.

13. Thrombusextraktionsvorrichtung nach Anspruch 1, wobei der Thrombuszerkleinerungsschneider (115) mit einem zentralen Durchgangsloch in Kommunikation mit dem Thrombuszugloch (1131) versehen ist; ein Durchmesser des Mehrlumenschlauchs (121) kleiner ist als ein Durchmesser des Übertragungsschlauchs (112), ein Durchmesser des Thrombuszuglochs, ein Durchmesser des zentralen Durchgangslochs.

14. Thrombusextraktionsvorrichtung nach Anspruch 1, wobei eine Vielzahl von Sätzen der Thrombusextraktionsanordnungen vorhanden ist, und sich die Vielzahl von Sätzen der Thrombusextraktionsanordnungen durch den Übertragungsschlauch (112) und den Thrombusabsaugkopf (113) erstreckt, und jeder Satz der Thrombusextraktionsanordnungen in Bezug auf die Thrombusabsauganordnung beweglich ist, und die Vielzahl von Sätzen der Thrombusextraktionsanordnungen jeweils außerhalb verschiedener Führungsdrähte (200) mit einer Hülse versehen ist.

## Revendications

1. Dispositif d'extraction de thrombus (100) configuré pour retirer un thrombus le long d'un fil de guidage (200), le dispositif d'extraction de thrombus comprenant :
un ensemble d'aspiration de thrombus comprenant un tube extérieur (111), un tube de transmission (112), un élément d'entraînement (114) et une tête d'aspiration de thrombus (113), dans lequel le tube extérieur est manchonné à l'extérieur du tube de transmission, une extrémité du tube extérieur est reliée à la tête d'aspiration de thrombus, la tête d'aspiration de thrombus est dotée d'une fraise d'écrasement de thrombus (115) en son sein, la tête d'aspiration de thrombus est configurée pour aspirer et écraser le thrombus, les deux extrémités du tube de transmission sont connectées à la fraise d'écrasement de thrombus et à l'élément d'entraînement, respectivement, l'élément d'entraînement est configuré pour entraîner une rotation du tube de transmission ; et,
un ensemble d'extraction de thrombus configuré pour attirer le thrombus vers la tête d'aspiration de thrombus, l'ensemble d'extraction de thrombus comprenant un tube multi-lumière (121) et un ballonnet (122) fourni à une extrémité du tube multi-lumière, dans lequel le ballonnet présente un état dégonflé et un état gonflé rempli de liquide, le tube multi-lumière est doté d'une cavité de remplissage de liquide (1211) en son sein qui est configurée pour remplir le ballonnet de liquide et d'une cavité de fil de guidage (1212) en son sein qui est configurée pour permettre au fil de guidage de s'étendre à travers celle-ci, le tube multi-lumière s'étend à travers le tube de transmission et la tête d'aspiration de thrombus de manière séquentielle, de sorte que l'ensemble d'extraction de thrombus est mobile par rapport à l'ensemble d'aspiration de thrombus dans une direction axiale ;
dans lequel la tête d'aspiration de thrombus a une structure creuse, la fraise d'écrasement de thrombus est fournie dans la tête d'aspiration de thrombus et est manchonnée à l'extérieur du tube multi-lumière, une surface d'extrémité de la tête d'aspiration de thrombus est dotée d'un trou de traction de thrombus (1131) configuré pour permettre au tube multi-lumière de s'étendre à travers celui-ci et d'au moins un trou d'extraction de thrombus (1132) configuré pour aspirer le thrombus, une surface d'extrémité de la fraise d'écrasement de thrombus est dotée d'une lame, et le trou d'extraction de thrombus est en face de la lame.

2. Dispositif d' extraction de thrombus selon la revendication 1, dans lequel l' ensemble d'extraction de thrombus comprend en outre un premier élément de connexion (123), le premier élément de connexion est manchonné sur une extrémité du tube multi-lumière (121) à l'écart du ballonnet (122), le premier élément de connexion est doté d'un orifice de remplissage de liquide (1231) en communication avec la cavité de remplissage de liquide (1211), et d'un orifice de fil de guidage (1232) en communication avec la cavité de fil de guidage (1212).

3. Dispositif d' extraction de thrombus selon la revendication 2, dans lequel l' ensemble d'extraction de thrombus comprend en outre une tête conique (124) connectée à une extrémité du tube multi-lumière (121) à l'écart du premier élément de connexion (123), la tête conique a une cavité interne en communication avec la cavité de fil de guidage (1212), de telle sorte que le fil de guidage (200) peut s'étendre à partir de la tête conique.

4. Dispositif d'extraction de thrombus selon la revendication 3, dans lequel un diamètre de la tête conique (124) augmente progressivement en direction vers le premier élément de connexion (123).

5. Dispositif d'extraction de thrombus selon la revendication 1, dans lequel, à l'état dégonflé, le ballonnet (122) est attaché à une surface du tube multi-lumière (121).

6. Dispositif d'extraction de thrombus selon la revendication 1, dans lequel, à l'état gonflé rempli de liquide, le volume du ballonnet (122) augmente, de sorte qu'une surface du ballonnet est étroitement attachée à une paroi interne d'un vaisseau sanguin.

7. Dispositif d'extraction de thrombus selon la revendication 1, dans lequel le ballonnet (122) est manchonné à l'extérieur du tube multi-lumière (121), et une paroi latérale du tube multi-lumière est dotée d'un orifice de remplissage de liquide de ballonnet (1213) en communication avec la cavité de remplissage de liquide (1211) et un intérieur du ballonnet.

8. Dispositif d'extraction de thrombus selon la revendication 1, dans lequel un espace (1111) est formé entre le tube extérieur (111) et le tube de transmission (112), et l'espace est configuré pour évacuer le thrombus aspiré et écrasé par l'ensemble d'aspiration de thrombus.

9. Dispositif d'extraction de thrombus selon la revendication 1, dans lequel l'ensemble d'extraction de thrombus comprend en outre un fil de développement (125) fourni à l'extérieur du tube multi-lumière (121) et situé aux deux extrémités du ballonnet (122).

10. Dispositif d' extraction de thrombus selon la revendication 1, dans lequel l'ensemble d'aspiration de thrombus comprend en outre un deuxième élément de connexion (118) manchonné sur une extrémité du tube extérieur (111) à l'écart de la tête d'aspiration de thrombus (113), le deuxième élément de connexion est doté d'un orifice d'aspiration de thrombus (1181) et d'un orifice de tube de transmission (1182), l'orifice d'aspiration de thrombus est en communication avec un intérieur du tube extérieur, et l'orifice de tube de transmission est configuré pour permettre au tube de transmission de s'étendre à travers celui-ci, l'ensemble d'aspiration de thrombus comprend en outre un dispositif d'aspiration à pression négative (116), le dispositif d'aspiration à pression négative est doté d'un orifice d'aspiration (1181) en communication avec l'orifice d'aspiration de thrombus, le dispositif d'aspiration à pression négative est en outre doté d'un orifice d'évacuation relié à un sac de collecte (117).

11. Dispositif d'extraction de thrombus selon la revendication 1, dans lequel le trou de traction de thrombus (1131) est fourni à un centre de la surface d'extrémité de la tête d'aspiration de thrombus (113).

12. Dispositif d' extraction de thrombus selon la revendication 11, dans lequel il y a une pluralité de trous d'extraction de thrombus (1132), et la pluralité de trous d'extraction de thrombus sont régulièrement fournis le long d'une direction circonférentielle du trou de traction de thrombus (1131).

13. Dispositif d'extraction de thrombus selon la revendication 1, dans lequel la fraise d'écrasement de thrombus (115) est dotée d'un trou traversant central en communication avec le trou de traction de thrombus (1131) ; un diamètre du tube multi-lumière (121) est inférieur à un diamètre du tube de transmission (112), à un diamètre du trou de traction de thrombus, et à un diamètre du trou traversant central.

14. Dispositif d'extraction de thrombus selon la revendication 1, dans lequel il y a une pluralité d'ensembles des ensembles d'extraction de thrombus, et la pluralité d'ensembles des ensembles d'extraction de thrombus s'étendent à travers le tube de transmission (112) et la tête d'aspiration de thrombus (113), et chaque ensemble des ensembles d'extraction de thrombus est mobile par rapport à l'ensemble d'aspiration de thrombus, et la pluralité d'ensembles des ensembles d'extraction de thrombus sont manchonnés à l'extérieur de différents fils de guidage (200), respectivement.
